Europäisches Patentamt

European Patent Office

Office européen des brevets

(11) Veröffentlichungsnummer: **0 345 568**

**A2**

## (12) EUROPÄISCHE PATENTANMELDUNG

(21) Anmeldenummer: **89109545.7**

(22) Anmeldetag: **26.05.89**

(51) Int. Cl.4: **G01N 33/00**

(30) Priorität: **04.06.88 DE 3819128**

(43) Veröffentlichungstag der Anmeldung:
**13.12.89 Patentblatt 89/50**

(84) Benannte Vertragsstaaten:
**AT BE CH DE ES FR GB GR IT LI LU NL SE**

(71) Anmelder: **Conducta Gesellschaft für Mess-
und Regeltechnik mbH & Co.
Dieselstrasse 24
D-7016 Gerlingen b. Stuttgart(DE)**

(72) Erfinder: **Schuldt, Hans-Peter
Löwenzahnweg 21
D-7257 Ditzingen 5(DE)**

(74) Vertreter: **Otte, Peter, Dipl.-Ing.
Tiroler Strasse 15
D-7250 Leonberg(DE)**

(54) **Gasdetektionssystem.**

(57) Bei einem Gasdetektionssystem zur Erfassung des Gehalts von brennbaren, explosiblen und/oder toxischen oder sonstigen Gasen wird vorgeschlagen, eine zentrale Auswerteelektronik mit einer Vielzahl von Meßköpfen vor Ort lediglich über eine einzige gemeinsame Zweidrahtleitung zu verbinden und jeden Meßkopf vor Ort mit eigener Intelligenz (Mikroprozessor) auszubilden mindestens zur Teilaufbereitung der vom angeschlossenen Sensor gelieferten Werte, wobei an jeden Meßkopf mindestens zwei Sensoren verschiedener Art oder unterschiedlicher Meßbereiche und ein Temperatursensor angeschlossen sind und jeder Sensor eine Kennung aufweist, die Typ und Meßbereich des Sensors angibt und vom Meßkopf erfaßt wird, mit Durchführung einer bidirektionalen digitalen Kommunikation zwischen Auswerteelektronik und den Meßköpfen nach deren Addressierung und mit der Stromversorgung des Meßkopfes über die Zweidrahtleitung.

Fig.1

## Gasdetektionssystem

Stand der Technik

Die Erfindung geht aus von einem Gasdetektionssystem nach dem Oberbegriff des Anspruchs 1.

Gasdetektionssysteme, bei denen also eine Vielzahl von das Vorhandensein von Gasen und deren jeweilige Menge erfassende Sensoren mit einer gemeinsamen Zentrale zur Auswertung verbunden sind, sind in vielfältiger, wenn auch komplizierter und umständlicher Form bekannt.

So ist ein insbesondere zur Überwachung von ausgedehnteren Garagen oder Tunneln geeignetes System so ausgebildet, daß an den verschiedensten Stellen Gasentnahmeköpfe vorgesehen sind, die über mechanische Schlauchverbindungen, die unter Umständen mehrere hundert Meter und darüber hinaus betragen müssen, mit einer gemeinsamen Auswertezentrale verbunden sind, wobei durch zyklisches, üblicherweise über angesteuerte Magnetventile bewirktes Umschalten von einem Schlauch zum anderen dem allerdings in der Zentrale dann nur einmal erforderlichen Sensor von den verschiedenen Entnahmestellen das dort vorhandene Gasgemisch zur Analyse zugeführt wird. Abgesehen von dem enormen mechanischen Aufwand, der nicht nur bei der Erstmontage, sondern auch nachfolgend zur Überwachung getrieben werden muß, beispielsweise zur Vermeidung, daß bestimmte Schläuche abknicken, ist ein solches Meßverfahren auch besonders langwierig, weil sich durch die stets dazwischenliegende notwendige Spülung der Schläuche sehr hohe Totzeiten ergeben, bis der Gassensor wieder mit dem aktuellen Meßgas versorgt werden kann. Ein solches System benötigt eine Vielzahl mechanisch beweglicher Teile, eine besonders hohe Pumpenleistung, eine häufige Wartung und verbindet diese Umstände mit dem Nachteil, daß bei der Messung keine Redundanz auftritt, eine Selbstüberwachung des Systems ausgeschlossen ist und Zykluszeiten im Bereich bis zu 30 Minuten und darüber liegen können.

Es ist daher auch schon bekannt, ein Gasdetektionssystem so aufzubauen, daß an den jeweils einer Überwachung oder Messung zu unterwerfenden Stellen jeweils ein mit einem Sensor bestückter Meßkopf angeordnet und über eine 5-adrige Leitung, gegebenenfalls auch nur über eine 3-adrige Leitung mit der Auswerteelektronik verbunden ist. Bei einer 5-adrigen Leitung dienen zwei der Leitungen zum Heizen des Sensors oder zu dessen Stromversorgung, zwei andere Leitungen dienen der Meßwertübertragung vom Meßkopf zur Auswerteelektronik und eine fünfte Leitung stellt die Masseverbindung her. Bei einer 3-adrigen Verbindung übernimmt die Masseleitung gleichzeitig auch die jeweils einen Verbindungsanschlüsse der beiden anderen Leitungsverbindungen und ersetzt immer eine Leitung. Ist an der gleichen Stelle oder, wie bei solchen Systemen üblicherweise beabsichtigt, an einer anderen Stelle eine weitere Gasanalyse erforderlich, dann müssen zur Detektion der zweiten Gaskomponente oder von Gasgemischen an anderen Stellen jeweils identische parallele Systeme mit Meßkopf und eigenem Verbindungskabel zur Auswerteelektronik geführt werden, d.h. die System- und Montagekosten sind erheblich.

Problematisch ist ferner bei solchen bekannten Gasdetektionssystemen, daß eine Mischbestückung nicht möglich ist wegen unterschiedlicher Meßkopfversorgungsspannungen, Empfindlichkeiten und Anschlüssen. Ferner verlangen diese Systeme nach der Montage und Installation einen individuellen Abgleich der Auswerteelektronik bis zum Meßkopf und umständliche Kalibrierungsschritte, auf die noch eingegangen wird.

Bestimmte Meßköpfe erfordern hohe Versorgungsströme, andere Meßköpfe weniger; was auch von der Art des Sensors und der zu überwachenden Gase abhängt, ob also auf brennbare, toxische, explosible oder sonstige Gase zu überwachen ist, wobei die vom Meßkopf herrührenden, stets sehr kleinen analogen Signalspannungen für die hohe Störspannungsempfindlichkeit verantwortlich sind, die solchen Systemen eigen ist. Daher ist es üblich, die vielen, von den einzelnen Meßköpfen zur Auswerteelektronik geführten Verbindungsleitungen, seien sie 3- oder 5-adrig, abgeschirmt zu verlegen. Üblicherweise verfügt jeder Meßkopf dabei über ein eigenes Netzteil, welches den an ihn angeschlossenen Sensor versorgt. Wegen der vielen unterschiedlichen Anforderungen, Meßbereiche, Arten von zu analysierenden Gasen u. dgl. ist es bisher nicht gelungen, hier Vereinheitlichungen im Sinne von Vereinfachungen einzuführen.

Zwar ist es bekannt, bei bestimmten chemischen Sensoren, nämlich solchen mit geringem Stromverbrauch den Meßkopf mit einem schwachen Versorgungsstrom zu beaufschlagen und über die gleiche Leitung ein Meßsignal ruckzuübertragen, wobei, um hier auch Zahlen zu nennen, der Versorgungsstrom beispielsweise bei maximal 4 mA liegt, während das Meßsignal im Bereich zwischen 4 ... 20 mA liegen kann.

Bei diesen bekannten Systemen ist insbesondere auch die sehr häufig notwendige Kalibrierung der Sensoren besonders umständlich, wobei eine solche Kalibrierung auf jeden Fall stets bei einem Austausch des Sensors, der auch nur immer an seinen bestimmten, ihm zugeordneten Meßkopf angeschlossen werden kann, erforderlich wird.

Eine solche Kalibrierung läuft z.B. so ab, daß sich eine Wartungsperson in der Zentrale, also bei der Auswerteelektronik befindet, während sich eine andere Wartungsperson mit Prüfgasflaschen entsprechender Konzentration vor Ort, also am jeweiligen Sensor befindet und dann diesen Sensor mit einem entsprechenden Prüfgas beaufschlagt. Die Verständigung zwischen der Wartungsperson in der Zentrale und dem Mann vor Ort erfolgt über Gegensprech-Funkgeräte, wobei nach Beschickung eines jeweiligen Sensors mit dem Prüfgas in hinreichender Konzentration bis zur Erzielung eines Gleichgewichts dieser Umstand dann zusammen mit der Art des Prüfgases der Wartungsperson in der Zentrale mitgeteilt wird; diese nimmt dann an den entsprechenden Einstellpotentiometern in der Zentrale die Eichung vor, vorausgesetzt, daß nicht in der Zwischenzeit aus irgendwelchen Gründen eine Konzentrationsänderung aufgetreten ist. Ein solches Kalibrierverfahren ist langwierig und auch störanfällig, da eine 2-Mann-Kalibrierung notwendig ist. Kalibrierfehler treten daher häufig auf.

Weitere Probleme beim bekannten Stand der Technik bestehen darin, daß die abgeschirmten Adern auf einer maximalen Länge von höchstens 1500 m in etwa verlegt werden können, wegen des hohen Meßkopfstroms und der Störanfälligkeit der kleinen Meßkopfsignale. In diesem Zusammenhang ist es noch erforderlich, einen Abgleich des Meßkopf-Brückenstroms in Abhängigkeit zur Leitungslänge vorzunehmen, desgleichen natürlich Abgleich auf Nullpunkt und Empfindlichkeit in Verbindung mit den hohen Installationskosten.

Da den bekannten Gasdetektionssystemen jede "Vorort elektronik" fehlt - dieser Begriff wird erst durch vorliegende Erfindung eingeführt -, benötigt man ferner neben dem Abgleichpotentiometer für den Brückenstrom in Abhängigkeit zur Leitungslänge ein Abgleichpotentiometer für Nullpunkt, ein Abgleichpotentiometer für Empfindlichkeit (Meßkopfsignalspannung), Abgleichpotentiometer für ersten und zweiten Alarm, Prüfbuchsen für Brückenstrom und Meßkopfsignal (Alterung), einen individuellen, manuellen Meßkopfsensorabgleich mit externem Meßinstrument, wobei Mischbestückung mit unterschiedlichen Meßkomponenten im Baugruppenträger nicht möglich ist.

Der Erfindung liegt daher die Aufgabe zugrunde, hier Abhilfe zu schaffen und die bekannten Gasdetektionssysteme dahingehend zu verbessern, daß mit geringstem Aufwand Meßköpfe an beliebiger Stelle angeordnet sind, die gleichzeitig, also parallel mehrere Gase in ihrem Gehalt bestimmen und mit der Auswerteelektronik in der Zentrale voll korrespondenzfähig sind, daß also mit anderen Worten neben der Übertragung der Versorgungsspannung an dem jeweiligen Meßkopf eine bidirektionale Signalübertragung zwischen Meßkopf und der zentralen Auswerteelektronik möglich ist.

## Vorteile der Erfindung

Die Erfindung löst diese Aufgabe mit den kennzeichnenden Merkmalen des Hauptanspruchs und hat den Vorteil, daß durch die Aufteilung der Vor-Ort-Apparatur in einen intelligenten, beispielsweise selbst mit einem Mikroprozessor ausgerüsteten Meßkopf mit Aufnahmemöglichkeiten für mindestens zwei Sensoren neben einem üblicherweise stets vorhandenen weiteren Temperatursensor ein bidirektionaler digitaler Nachrichtenverkehr, also eine Digitalsignalübertragung zwischen der Elektronikzentrale und dem Meßkopf in beiden Richtungen möglich ist, so daß jede Störanfälligkeit in diesem Bereich nahezu beseitigt ist.

Ferner ist es nunmehr möglich, an eine einzige Zweidrahtleitung eine im Grunde beliebige Anzahl von Vor-Ort-Apparaturen, jeweils bestehend aus Meßkopf mit mindestens zwei Gassensoren und Temperatursensor, anzuschließen, wobei auch beliebige Reihen/Parallelschaltungen möglich sind.

Lediglich für den Fall, daß bei sogenanntem Ex-Betrieb der Leitungsstrom bestimmte Werte nicht übersteigen darf, kann eine Begrenzung der Zahl der angeschlossenen Meßköpfe mit ihren Sensoren vorgenommen werden, wobei dann eine informations-technische Verknüpfung der zur Elektronikzentrale geführten Leitungen innerhalb derselben erfolgen kann.

Vorteilhaft ist ferner die hohe Meßsicherheit, die geringe Wartung und die kostengünstige und einfache Installation, wobei noch besonders vorteilhaft ist, daß die Meßköpfe mit beliebigen Sensoren bestückt werden können, also entweder bei erforderlichem Austausch eines oder beider oder mehrerer Sensoren an einem Meßkopf wegen Alterung u. dgl. identische Sensoren wieder angebracht werden können, oder es können auch Sensoren für die Erfassung anderer Gase und/oder mit anderen Meßbereichen dem Standard- oder Universalmeßkopf zugeordnet werden. Der Grund hierfür liegt darin, daß zwei verschiedene Erkennungssysteme in das erfindungsgemäße Gasdetektionssystem eingearbeitet sind, nämlich einmal eine Kennung zwischen den jeweiligen, an einen Meßkopf angeschlossenen Sensoren und die Adressierfähigkeit der Sensoren von der Auswerteelektronik in der Zentrale, so daß die eine vorhandene Zweidrahtleitung problemlos für die Erfassung der Meßdaten einer Vielzahl von Vor-Ort-Apparaturen bei kürzesten Zykluszeiten durch die zentrale Auswerteelektronik eingesetzt werden kann.

Neben der wahlweisen Bestückung des jeweiligen Meßkopfes ermöglicht die Erfindung eine dynamische Temperaturkorrektur, eine automatische Lebensdauerkontrolle durch ein spezielles Wartungssignal, eine automatische Meßkompensation sowie Bereichsabgleich aufgrund der Sensorkennung sowie aus dem gleichen Grund bei Nachbestückung mit gleichen oder anderen Sensoren (Sensortausch) den Wegfall jeglicher Abgleicherfordernisse.

Als 2-adriges Verbindungskabel können Installations- oder Telefonkabel eingesetzt werden, wobei Übertragungsentfernungen bis zu 5000 m möglich sind; im speziellen Ausführungsbeispiel, welches die Erfindung nicht einschränkt, erfolgt die Übertragung auf dem Telefonkabel als Digitalbus bidirektional für 3 Meßwerte (bei Gassensoren und Temperaturmeßwert) und zusätzlichen 8 Statusignalen, bei einer Übertragungsrate von 1200 Baud. Dabei erfolgt die Übertragung der Sensorkennung durch die Vor-Ort-Elektronik, z.B. einen Mikroprozessor im Meßkopf, wobei auch eine automatische Sensor-Lebenszeitkontrolle erfolgt.

Ein besonderer Vorteil vorliegender Erfindung besteht noch darin, daß die Vor-Ort-Elektronik nunmehr eine automatische Systemkalibrierung mit einer speziellen Kalibrierkappe möglich macht, ohne daß ein manueller Abgleich erforderlich ist. Durch entsprechende Speicherung von Werten in der zentralen Auswerteelektronik, beispielsweise in einem EPROM erfährt die zentrale Auswerteelektronik aus der Kennung des an den jeweiligen, adressierten Meßkopf angeschlossenen Sensors dessen Eigenschaften, also welche Gase er erfassen kann und welche Meßbereiche er aufweist, wobei diese Kennung beispielsweise in einer binären Verschlüsselung körperlich beim Einsetzen oder Anbringen des Sensors an den intelligenten Meßkopf von diesem erfaßt und auch für die Abfrage durch die zentrale Meßelektronik zur Verfügung gehalten wird.

Durch die in den Unteransprüchen aufgeführten Maßnahmen sind vorteilhafte Weiterbildungen und Verbesserungen des im Hauptanspruch angegebenen Gasdetektionssystems möglich. Besonders vorteilhaft ist die Ausbildung der Kennung jedes Sensors in Form einer binären Verschlüsselung von Steckkontakten, wobei lediglich, je nach gewünschter Kennung im binären System, durch Verbindung von Lötpunkten Brücken hergestellt werden. Diese Kennung wird ab Werk vor Auslieferung angebracht, wodurch der beliebige Austausch vor Ort später möglich ist, da von der Intelligenz des Meßkopfes an die zentrale Auswerteelektronik automatisch der Typ des Sensors sowie seines Meßbereichs gemeldet wird. Es ist dann möglich, daß auf einem geeigneten Anzeigedisplay in der zentralen Warte automatisch die richtige Konzentration und die jeweils gemessene Komponente angezeigt wird. Verglichen mit dem bekannten umständlichen Meßverfahren über Schlauchverbindungen oder mehradrigen, abgeschirmten Kabelleitungen zu jedem Meßkopf gelingt es daher der Erfindung, eine mit Bezug auf den bekannten Stand der Technik umwälzende Vereinfachung und Meßsicherheit bei der Erfassung des Vorhandenseins von Gasen und deren Konzentration herbeizuführen.

Zeichnung

Ausführungsbeispiele der Erfindung sind in der Zeichnung dargestellt und werden in der nachfolgenden Beschreibung näher erläutert. Es zeigen:

Fig. 1 schematisiert die verschiedenen Verbindungsmöglichkeiten zwischen den einzelnen Meßköpfen und der zentralen Auswerteelektronik;

Fig. 2 den schrittweisen Aufbau von verschiedenen Sensorbaugruppen und deren Zuordnung zu jeweiligen Meßköpfen und

Fig. 3 ebenfalls schematisiert eine Kalibriereinrichtung, wie sie im Rahmen vorliegender Erfindung nunmehr möglich geworden ist.

Beschreibung der Ausführungsbeispiele

Der Grundgedanke vorliegender Erfindung besteht darin, im Bereich der Gasanalyse eine Vor-Ort-Korrespondenzfähigkeit zwischen Sensor/Meßkopf einerseits und zentraler Auswerteelektronik andererseits einzuführen, wobei der Meßkopf mit einer eigenen Intelligenz, nämlich einem Einchiprechner, Mikroprozessor o. dgl. ausgestattet wird und die Signalübertragung in beiden Richtungen digital erfolgt, und wobei gleichzeitig hiermit durch nur eine einzige, 2-adrige Verbindungsleitung von im Grunde beliebigem Aufbau (auch und insbesondere Telefonkabel) der Datenaustausch zwischen den verschiedenen Meßköpfen und der zentralen Auswerteelektronik seriell aufeinanderfolgend durchgeführt wird, bei gleichzeitiger Speisung der Meßköpfe über die eine Übertragungsleitung mit dem erforderlichen Versorgungsstrom.

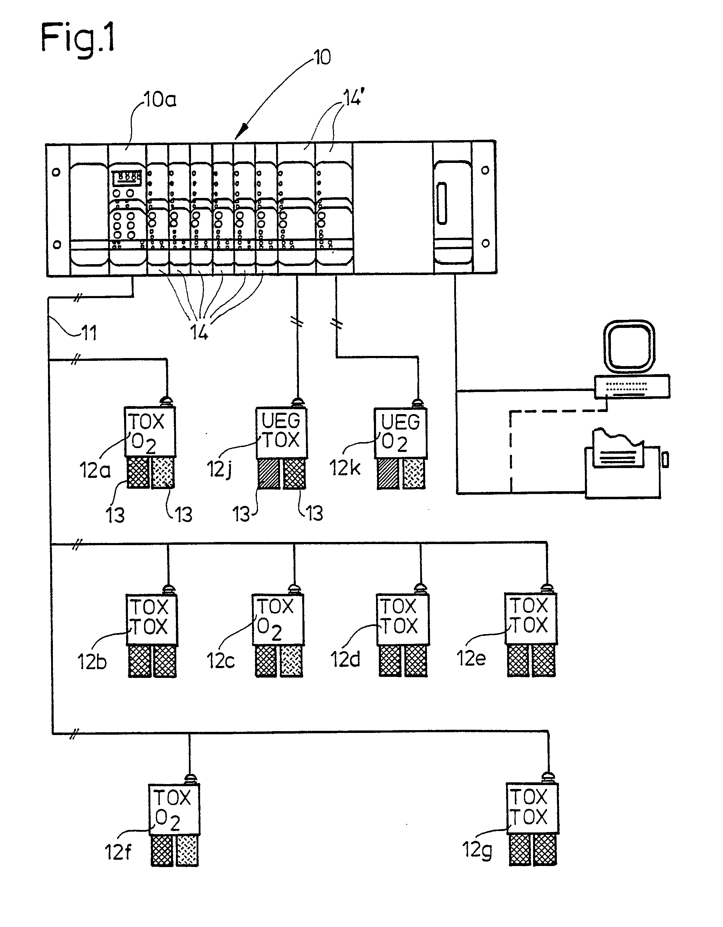

In Fig. 1 ist die zentrale Auswerteelektronik mit 10, eine von ihr ausgehende gemeinsame Zweidrahtleitung mit 11 und an diese Zweidrahtleitung angeschlossene Meßköpfe mit 12a, 12b ... bis 12g bezeichnet.

In Fällen sehr hohen Strombedarfs der Meßköpfe, im Bild mit 12j und 12k, können auch getrennte Verbindungsleitungen vorgesehen sein, deren digitale Signale durch geeignete Schaltungsmittel jedoch auch der zentralen Auswerteeinheit 10 zugeführt werden.

Jeder der Meßköpfe 12a bis 12k enthält einen eigenen Mikroprozessor und kann mit mehreren, bei dem dargestellten Ausführungsbeispiel mit zwei Sensoren unterschiedlichster Funktion bestückt werden zur Gasanalyse. So können im speziellen Fall, was an den Meßköpfen angegeben ist, diese nur toxische, explosible oder Sauerstoffgemische allein bestimmen; es können aber auch bei der dargestellten Bestückung mit zwei Sensoren jede gewünschte Kombination, zum Beispiel ein toxisches und ein explosibles Gasgemisch oder zwei toxische Gasgemische unterschiedlichster Komponenten oder die Kombination von Sauerstoffbestimmung mit toxischen oder explosiblen Gasgemischen durchgeführt werden, wobei ergänzend hierzu noch eine Vielzahl von Sensoren zur Bestimmung des gleichen Gases, jedoch mit unterschiedlichen Meßbereichen hinzu kommen können.

In der Kurzform der von den Sensoren zu erfüllenden Aufgabe sind in Fig. 1 Sensoren bzw. Meßköpfe zur Messung von toxischen Gasen mit TOX, zur Messung von explosiblen Gasen oder Gasgemischen mit UEG, zur Messung von Sauerstoff oder Sauerstoffmangel mit O2 bezeichnet, was nur einige der Meßmöglichkeiten angibt, die die Erfindung zu leisten imstande ist.

Die Sensoren sind in Fig. 1 durchgehend mit dem Bezugszeichen 13 versehen, unabhängig davon, welchen Typ sie darstellen oder welche Aufgaben (Meßbereich) sie erfüllen. Mit Vorzug stets ergänzend noch an jedem Meßkopf vorhandene Temperatursensoren sind der Übersichtlichkeit halber nicht dargestellt.

In der zentralen Auswerteelektronik ist eine Hauptrechenschaltung mit umfassenden Speichermöglichkeiten mit 10a im Einschubbereich bezeichnet; von dieser geht eine der Zweidrahtleitungen 11 aus, die mit den Meßköpfen 12a bis 12g verbunden ist. Diese Hauptrechenschaltung verfügt für jeden der an sie angeschlos senen Meßköpfe 12a - 12g über entsprechende Informationen, so daß sie diese Meßköpfe einer gewünschten vorgegebenen Abfolge nach (hier gibt es verschiedene Verfahren, nach welchen auch prioritätsberechtigte Meßköpfe bevorzugt abgefragt werden, auf die aber in diesem Zusammenhang nicht weiter eingegangen zu werden braucht, da dies für sich gesehen bekannt ist) mit einem vorgegebenen Code adressiert, also anspricht, wodurch sich dann eine bidirektionale serielle Übertragung eines Protokolls anschließt. Jeder Meßkopf bildet mit Bezug auf die an ihn angeschlossenen Sensoren 13 eine Schnittstelle zur zentralen Auswerteelektronik; diese erhält vom entsprechenden Meßkopf beim normalen zyklischen Abfragedurchlauf (zunächst seien Kalibrierungsschritte nicht berücksichtigt) die jeweilige Sensorkennung S1 bzw. S2 für die angeschlossenen Gassensoren und S3 für den Temperatursensor übertragen und daraufhin entsprechende normierte Meßwerte in binär-codierter Form, die von der Meßkopfintelligenz zunächst auf der analogen Seite beispielsweise als Spannungswerte zwischen 0 bis 1 V aufbereitet und anschließend digitalisiert worden sind, ergänzt um die Berücksichtigung peripherer Bedingungen wie Temperaturkompensation, Kennlinienkorrektur, Linearisierung und sonstige weitere Korrekturen wie Feuchteabhängigkeit oder Luftdruckabhängigkeit.

Die Schnittstelle Meßkopf spricht mit der durchgegebenen Sensorkennung zunächst einen entsprechenden Speicherbereich in der Hauptrechenschaltung 10a der zentralen Auswerteelektronik an, so daß der anschließend, oder auch vorher, übermittelte jeweilige binär codierte, also in digitaler Form über die Leitung übertragene Meßwert dieses Sensors die zutreffende Einordnung und Bewertung erfährt.

Tatsächlich befinden sich im Bereich der zentralen Auswerteelektronik weitere Einschübe, die insgesamt mit 14 bezeichnet sind und die den jeweiligen Meßköpfen oder vorgesehenen Sensoren zugeordnet sind und an diese Einschübe leitet die Hauptrechenschaltung 10a den jeweils für einen betreffenden Sensor erfaßten Meßwert weiter, so daß dann der diesem Sensor zugeordnete Einschub in der Lage ist, an seinem Display die erfaßten Meßwerte in geeigneter Form anzuzeigen, und zwar zusammen mit sonstigen Anzeigemöglichkeiten wie beispielsweise Alarm-, Diagnose-, Störungs-, Kalibrierungs- und Betriebs-Zustandsanzeigen, üblicherweise Leuchtdioden oder sonstige Lampen. Die zentrale Auswerteelektronik mit ihrer Hauptrechenschaltung ist daher der Verwalter der eingehenden Daten und teilt diese über einen internen Bus an die Einschübe 14 mit, so daß hier wieder die Aufteilung entsprechend den Vor-Ort-Meßplätzen erfolgen kann, soweit dies gewünscht und erforderlich ist.

Es versteht sich, daß aufgrund der vielfältigen Möglichkeiten, die sich bei einer solchen Art der Datenerfassung und -verwaltung ergeben, auch sonstige Gesichtspunkte noch Berücksichtigung finden können, beispielsweise neben der dynamischen Erfassung der Umgebungstemperatur am Meßort auch Daten über die Lebenserwartung der einzelnen Sensoren, die sich problemlos aus der Betriebsdauer und den im Speicher der Hauptrechenschaltung niedergelegten Daten errechnen lassen. Es ist dann möglich, an den Einschüben entsprechende Anzeigen vorzunehmen, wenn zugeordnete Sensoren erschöpft sind.

Ferner kann die Anzeigetemperatur dem Betreiber, der die zentrale Auswerteelektronik mit ihren Einschüben in der Meßwarte natürlich ständig vor Augen hat, auch über evtl. auftretende Gefahrenzustände seiner Anlage vor Ort hinweisen, beispielsweise entstehender Brand/ Feuer, da der Temperatursensor natürlich auch solche Daten überträgt.

Es versteht sich, daß die Aufgaben, die je nach seiner Ausgestaltung oder seinen Beschränkungen von der Meßkopfelektronik, speziell von deren Mikroprozessor nicht wahrgenommen werden können, in der Verarbeitung der von den Sensoren übermittelten Daten dann unter Berücksichtigung gespeicherter Daten und sonstiger Korrekturen von der Hauptrechnerschaltung 10a durchgeführt werden. Die Aufteilung kann hier beliebig erfolgen, wobei zu berücksichtigen ist, daß die Hauptrechnerschaltung 10a mit ihren Speichermöglichkeiten auf jeden Fall darüber informiert ist, unter welchen Voraussetzungen der ihr jeweils übermittelte momentane Meßwert des Sensors zustandegekommen ist.

In der Meßwarte wird auf jeden Fall die Einsatz-Umgebungstemperatur des Meßkopfes mit angezeigt mit der Möglichkeit, die Temperaturabhängigkeit der Sensoren auch erst im Bereich der zentralen Auswerteelektronik zu kompensieren.

Im praktischen Aufbau sind an jedem Meßkopf (zunächst) 2 Sensorgehäuse montiert (für die Gasanalyse, wobei natürlich je nach Auslegung dieses Schnittstellenbereichs, der Speichermöglichkeiten und der Ausgestaltung der zentralen Auswerteelektronik auch noch weitere Sensoren angeschlossen werden können, wenn hierzu Bedarf besteht). So können beispielsweise, um hier mit Zahlen zu arbeiten, etwa 10 ihrem Typ nach verschiedene Sensoren vorgesehen sein, mit zum Beispiel 30 verschiedenen Meßbereichen. Im Fehlerfall oder auch nach Wahl des Betreibers können sämtliche Sensoren ihrem Typ und ihrem Meßbereich nach problemlos an beliebigen Meßköpfen zur Umstellung auf andere Gaskomponenten oder andere Meßbereiche getauscht werden, wobei die Mikroprozessor-Elektronik im Meßkopf zusammen mit der digitalen Übertragung der Signale in bidirektionaler Richtung einen solchen Sensortausch durch die spezielle, sensorbezogene Kennung problemlos ermöglicht.

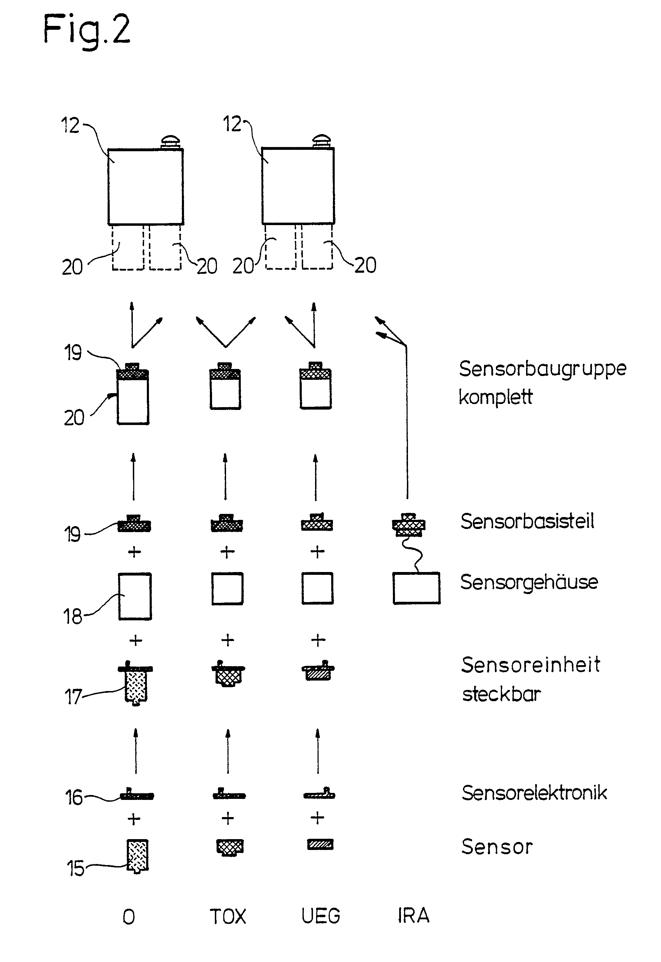

In Fig. 2 sind hierzu unterschiedliche Sensoraufbauten dargestellt, wobei jeweils unterhalb des Sensors dessen Eigenschaften ($O_2$, TOX, UEG, IRA, letzterer zur Infrarotgasanalyse) angegeben sind.

Das eigentliche Sensorelement 15 wird mit einer einfachen Sensorelektronik 16 verbunden und diese beiden Teile als Sensoreinheit 17 in ein Sensorgehäuse 18 mit zugeordnetem Basisteil 19 untergebracht. Es ergibt sich dann eine typische, untereinander jedenfalls im Bereich des Sensorbasisteils oder Sockels 19 einheitliche Sensorbaugruppe 20, die mit ihrem Sockel so, wie bei den darüber angeordneten Meßköpfen 12 gezeigt, in diese über geeignete Sockelsteckkontakte eingeführt und so an dieser auch befestigt werden kann, wodurch gleichzeitig die elektrischen Verbindungsanschlüsse vorgenommen werden. Wie weiter vorn schon erwähnt, ist wesentlich, daß am Sockel bzw. Sensorbasisteil 19 die Kennung jedes Sensors für den Meßkopf lesbar angeordnet ist; diese sensorbezogene Kennung wird an dem jeweiligen Sensor sofort in der Produktion angebracht, so daß vor Ort dann jeder beliebige Austausch möglich ist, denn die Schnittstelle Meßkopf meldet durch die von ihr erfaßte Kennung der zentralen Auswerteelektronik automatisch den Typ des Sensors sowie dessen Meßbereich.

Es ist vorteilhaft, bei jedem Abfragezyklus diese Codierung Typ und Meßbereich automatisch abzufragen und zu vergleichen, wobei dann im Speicher (vorzugsweise ein sog. EPROM) der zentralen Auswerteelektronik die entsprechenden Arbeitsroutinen je nach Sensorkennung abgelegt sind, so daß auf den entsprechend zugeordneten Anzeigedisplays automatisch die richtige Konzentration und die Komponente angezeigt wird. Es ist schon erwähnt, daß die Kennung bei einem bevorzugten Ausführungsbeispiel eines Sensors durch das Verbinden von elektrischen Kontaktpunkten durch Brücken im Sockelbereich vorgenommen wird; hierdurch kann der Mikroprozessor im Meßkopf die erforderlichen Angaben entnehmen und aus diesem Grund ist auch bei Nachbestückung ein Abgleich nicht mehr notwendig.

Ein weiterer Vorteil besteht darin, daß dieses Grund prinzip der Stromversorgung im Meßkopfbereich sowie der digitalen Meßwert- und Statussignalübertragung problemlos, jedenfalls bei Sensoren mit nur geringen Versorgungsstromansprüchen eigentliche Stromkreise nach der Schutzart EEx ib (für die Sensoren EEx di) ermöglicht; dort, wo unter Umständen zu hohe Ströme die Speisung aus einer einzigen Zweidrahtleitung 11 (Fig. 1) nicht zulassen, können mit den entsprechenden Einschüben 14 über eigene Leitungen verbundene Meßköpfe 12j und 12k gegebenenfalls vorgesehen sein.

Es versteht sich, daß über die interne Busleitung in der zentralen Auswertelektronik 10 und entsprechend vorgesehene Interface-Schaltungen der Anschluß übergeordneter Rechensysteme bzw. Drucker oder sonstiger Rechner möglich ist. So können die Meß- und Statuswerte sowie andere Betriebszustände nach beliebigen Kriterien ausgedruckt und festgehalten werden.

Die bisher erläuterte Grundkonzeption macht noch eine weitere, besonders vorteilhafte Ausgestaltung möglich, nämlich die im Bereich der Meßwarte vollkommen automatisch durchzuführende Kalibrierung der Gassensoren, wobei sämtliche Fehlbedienungsmöglichkeiten und Irrtümer ausgeschaltet werden, da lediglich noch vor Ort eine Bedienungsperson am jeweiligen Sensor direkt tätig zu werden braucht.

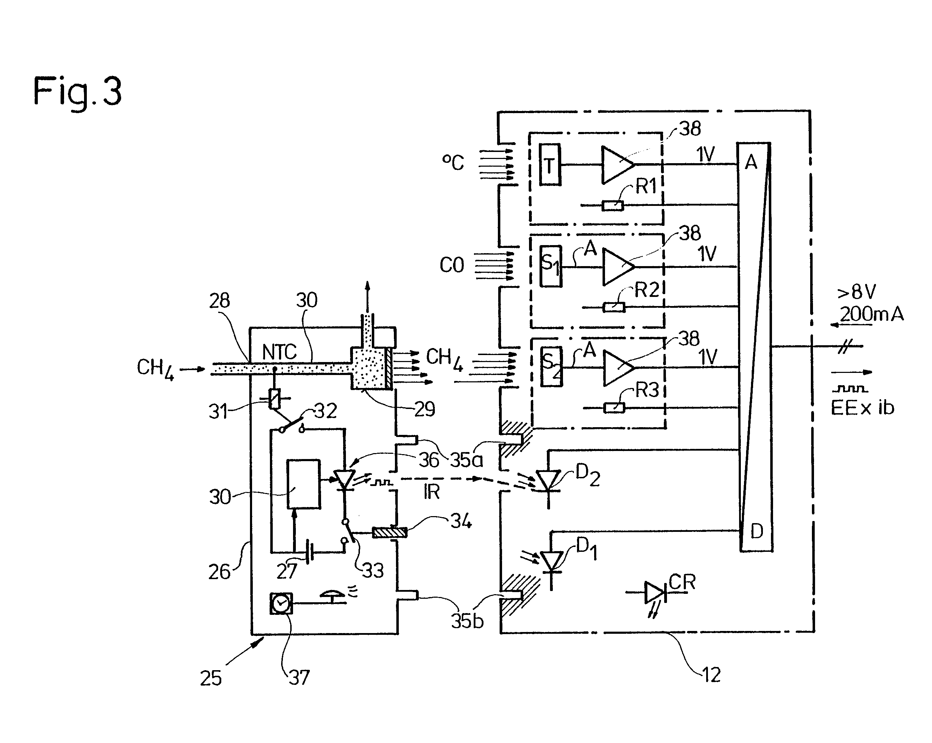

Die Darstellung der Fig. 3 zeigt schematisiert das Grundprinzip zur Durchführung der Kalibrierung.

Es ist eine Kalibriereinrichtung 25 in der praktischen Ausführungsform einer Kalibrierkappe vorgesehen, die über jedes mit dem Meßkopf fest verbunden bleibende Sensorgehäuse gestülpt wird. Die Kalibrierkappe 26 verfügt über eine eigene Stromversorgung wie beispielsweise eine Batterie 27, einen äußeren Gasanschluß 28, ein kappenförmiges Gehäuse, welches bei 29 angedeutet dargestellt ist, mit einer solchen Ausbildung und Form, daß es über den gassensitiven Bereich der Sensorbaugruppe (abgedichtet) gestülpt werden kann, und einen Elektronikbereich 30, der vom Meßkopf auf zunehmende Kalibriersignale erzeugt.

Gassensoren können nach Nullpunkt und Empfindlichkeit (Steigung) eine Kalibrierung erforderlich machen; entsprechend ist an den Gasanschluß 28 ein Prüfgas vorgegebener Konzentration jeweils anzuschließen. In der Leitung 30 vom äußeren Gasanschluß 28 zur Kappe 29 befindet sich ein Strömungsmesser, vorzugsweise in Form eines NTC-Widerstands, der die vorhandene Eichgasströmung feststellt und, beispielsweise über ein zwischengeschaltetes Relais 31 einen ersten Schal -ter 32 betätigt. Durch das mechanische Aufstecken der Kalibrierkappe auf das Sensorgehäuse kann ein zweiter Schalter 33 ebenfalls betätigt werden, und zwar beispielsweise durch einen einfachen Schaltstift 34, der beim Aufstecken eingedrückt wird. Hier können natürlich auch andere Schaltmöglichkeiten vorgesehen sein, beispielsweise eine manuelle Schalterbetätigung durch die Bedienungsperson nach dem Aufstecken der Kalibrierkappe. Die Kalibrierkappe kann noch Führungsmittel 35a, 35b aufweisen zum ordnungsgemäßen Verbinden mit den Sensoren und dem Meßkopf, wobei, wie es sich versteht, auch die ansonsten vollautomatisch ablaufende Kalibrierung beider oder mehrerer Gassensoren am Meßkopf sowie auch des Temperatursensors möglich ist. In Fig. 3 sind die hier vorhandenen zwei Sensoren mit S1 und S2 sowie der Temperatursensor mit T bezeichnet.

Sind beide Schalter 32 und 33 geschlossen, dann erzeugt ein geeigneter Sender 36 im Bereich der Kalibrierkappe 25, vorzugsweise eine mit einem geeigneten binär-codierten Signal oder einfach mit einer vorgegebenen Frequenz (1 kHz) beaufschlagte Leuchtdiode ein Signal, beispielsweise dieses 1 kHz-Signal (im Infrarotbereich beispielsweise) und beaufschlagt hiermit eine Empfängerdiode D2 im Meßkopf, die für den Sensor S2 zuständig ist.

Liegt also die richtige Eichgasströmung vor, dann erzeugt die Kalibrierkappe das Kalibriersignal (zunächst für einen ersten Sensor S2); der Mikroprozessor des Meßkopfes 12 erkennt dieses Kalibriersignal und überträgt es an die zentrale Auswerteelektronik, so daß die Auswerteelektronik in Korrespondenz mit diesem speziellen Meßkopf jetzt in eine "Kalibrierphase" übergeht, wobei die zentrale Auswertelektronik mit einem Rücksignal an dem Meßkopf zunächst die Erkennung und die Bereitschaft zur Kalibrierung meldet. Dieses Kalibrier-Rückbestätigungssignal wird am Meßkopf in geeigneter Weise angezeigt, und zwar über eine Leuchtdiode CR, was bedeutet, daß das Funktionssignal "Kalibrierung" nunmehr aufleuchtet und auch die Bedienungsperson weiß, daß das gesamte Prozessorsystem die Kalibrierwerte akzeptiert und übernimmt.

Hier ist noch auf etwas hinzuweisen: Da die zentrale Auswerteelektronik erkennen muß, ob es sich um ein sogenanntes Nullgas zur Nullpunktbestimmung bzw. um ein Prüfgas zur Empfindlichkeitsbestimmung handelt, interpretiert die zentrale Auswerteelektronik Gaskonzentrationen von beispielsweise weniger als 10%, die sie während der "Kalibrierphase" erfaßt, als Eich- oder Nullgas zur Nullpunktbestimmung und Konzentrationen über z.B. 20% zur Empfindlichkeitsbestimmung, wobei das Prüfgas dann eine vorgegebene Konzentration von beispielsweise 50% des Meßbereichsendwertes haben kann. Aufgrund der in der zentralen Auswertelektronik gespeicherten Werte und Daten ist diese über die Prüfgaszusammensetzung, mit denen sie konfrontiert wird, informiert und kann dann automatisch die entsprechenden Eichvorgänge durchführen.

Die Eichung läuft im Prinzip so ab, daß die zentrale Auswerteelektronik bei den während der Kalibrierphase eingehenden Meßwerten darauf achtet, wann das eingehende Signal seine Konzentration nicht mehr ändert, also das "Konzentrationssignal" in einer bestimmten Zeit gegen Null bzw. gegen einen sonstigen stabilen Zustand läuft. Dies erkennt die zentrale Auswerteelektronik als Hinweis darauf, daß der Sensor im eingeschwungenen Zustand die Prüfgaskonzentration übermittelt und korrigiert, gegebenenfalls entsprechend die in ihr gespeicherten Angaben für Nullpunkt und Steilheit (Empfindlichkeit) dieses betreffenden Sensors, wobei dann der Kalibriervorgang vorzugsweise nicht durch das Bedienungspersonal, sondern auf Wei sung der zentralen Auswerteelektronik automatisch beendet wird. Die zentrale Auswerteelektronik nimmt nach Beendigung des Kalibriervorgangs das Funktionssignal "Kalibrierung" (Leuchten der Diode CR) wieder weg oder läßt diese intermittierend aufleuchten, so daß die Bedienungsperson den Kalibriervorgang für diesen Sensor als beendet erkennt und die Kalibrierkappe abziehen kann für die Kalibrierung des nächsten Sensors an diesem Meßkopf. Hierzu wird die Kalibrierkappe 25 auf diesen anderen Sensor aufgesteckt, das Kalibriersignal wird von der Kalibrierkappe erneut erzeugt und gelangt nunmehr auf einen zweiten Empfänger in Form einer Photodiode D1, wobei beispielsweise nunmehr eine Frequenz von 2kHz erzeugt werden kann. Es ist aber auch möglich, dieses insofern dann unterschiedliche Kalibriersignal dem gleichen Empfänger im Meßkopf zuzuführen, wobei es auch möglich ist, die Kalibrierkappe sofort so auszubilden, daß sie über zwei angrenzende Kappen verfügt, die die jeweiligen Sensoren am Meßkopf aufeinander folgend mit den Prüfgasen beaufschlagen.

Die Kalibrierkappe kann auch über eine Schaltung 37 zur Erzeugung eines eigenen Zeittaktes verfügen. Ausdrücklich sei darauf hingewiesen, daß die Kalibrierung durch Einstellen entsprechender Schaltungen im Bereich des Meßkopfes bzw. entsprechender Speicherung der neuen, aus der Kalibrierung entstandenen Daten im Meßkopf durchgeführt werden kann; dies ist auch insofern vorteilhaft, als der Meßkopf ohnehin über lediglich durch das Verstärkerzeichen in Fig.3 angedeutete Elektronikmittel 38, zugeordnet jedem Sensor verfügt, um als Interface insofern standardisierte Meßsignalgrößen zwischen 0 und 1 V in binärcodierter Form auf die Leitung geben zu können.

Zusammengefaßt: Das Gasdetektionssystem mit Meßkopf und zentraler Auswerteelektronik erkennt automatisch, daß kalibriert wird, wobei der Kalibriervorgang automatisch überwacht und die Kalibrierwerte automatisch übernommen werden mit ständiger Information der Bedienungsperson, so daß auch hier mit einem Schlage die Probleme bei der stets notwendigen Kalibrierung von Gassensoren, wie sie nach dem Stand der Technik auftreten, beseitigt sind. Die mit der Kalibrierung beauftragte Bedienungsperson braucht lediglich noch die Kalibrierkappe an das Sensorgehäuse zu halten und das jeweils zutreffende Prüfgas zuzuführen. Sämtliche anderen Vorgänge laufen automatisch aufgrund der bidirektionalen Korrespondenzfähigkeit des Systems ab, so daß Fehler vollkommen ausgeschlossen sind.

Alle in der Beschreibung, den nachfolgenden Ansprüchen und der Zeichnung dargestellten Merkmale können sowohl einzeln als auch in beliebiger Kombination miteinander erfindungswesentlich sein.

**Ansprüche**

1. Gasdetektionssystem, insbesondere zur Erfassung des Gehalts von brennbaren, explosiblen und/oder toxischen Gasen, zur Bestimmung des Sauerstoffgehalts oder -mangels u. dgl., mit einer Anzahl von Gassensoren vor Ort, die mit einer Auswertezentrale (zentrale Auswerteelektronik) verbunden sind, dadurch gekennzeichnet, daß

a) die zentrale Auswerteelektronik (10) mit einer Vielzahl von Meßköpfen (12; 12a, 12b ... 12g) vor Ort lediglich über eine einzige gemeinsame Zweidrahtleitung (11) oder einige wenige Zweidrahtleitungen verbunden ist,

b) daß jeder Meßkopf vor Ort eine eigene Intelligenz (Mikroprozessor) aufweist mindestens zur Teilaufbereitung (Linearisierung, Kennlinienkorrektur, Temperaturkompensation, Kalibrierung, Standardisierung) der vom Sensor gelieferten Werte,

c) daß an jeden Meßkopf (12; 12a, 12b, 12c ... 12g) ein oder mehrere Sensoren verschiedener Art oder unterschiedlicher Meßbereiche, gegebenenfalls mit zusätzlichem Temperatursensor, angeschlossen sind,

d) daß zur Verbindung von Sensor und Meßkopf an dem jeweiligen, jedoch seinem Typ und seinem Meßbereich nach beliebigen Sensor, eine Kennung angeordnet ist, die Typ, Meßgaskomponente und Meßbereich des Sensors (13) angibt und vom Meßkopf (12) erfaßt wird und daß

e) zwischen der zentralen Auswerteelektronik (10) und den Meßköpfen über die Zweidrahtverbindungsleitung(en) (11) eine bidirektionale, digitale Kommunikation entsprechend einem der an sich bekannten Mehrpartnerverfahren durchgeführt wird als auch die Gleich- oder Wechselstromversorgung des Meßkopfes erfolgt.

2. Gasdetektionssystem nach Anspruch 1, dadurch gekennzeichnet, daß die mit den Meßköpfen (12, 12a bis 12g) zu verbindenden Sensoren (13) jeweils vereinheitlichte Sockelanschlüsse und die Meßköpfe entsprechend ausgebildete Aufnahmeöffnungen (Stekker und Buchse) aufweisen und daß die am jeweiligen Sensor angebrachte Kennung in Form von elektrischen Kontakten für den Meßkopf erkennbar ausgebildet ist.

3. Gasdetektionssystem nach Anspruch 1 oder 2, da durch gekennzeichnet, daß am jeweiligen Sensor dessen Typ, dessen Meßgaskomponente und Meßbereich in binärcodierter Form angebende Lötkontaktbrücken für die Meßkopf-Sockelfassung identifizierbar angeordnet sind.

4. Gasdetektionssystem nach einem der Ansprüche 1-3, dadurch gekennzeichnet, daß mindestens im Bereich der zentralen Auswerteelektronik Speichermittel (EPROM) vorgesehen sind mit bestimmten Sensoren nach Typ, Meßgaskomnponente und Meßbereich zugeordneten Daten derart, daß die Auswerteelektronik nach Empfang der Sensorkennung über den Meßkopf, entsprechenden Statussignalen und den jeweiligen Meßwerten diese auswerten und verarbeiten kann.

5. Gasdetektionssystem nach Anspruch 4, dadurch gekennzeichnet, daß die von der zentralen Auswerteelektronik für jeden Sensor erfaßten und umgesetzten Meßdaten (über einen internen Bus) in entsprechender Aufteilung separaten Einschüben mit jeweiligen Anzeigedisplays für die verschiedenen Betriebs- und Funktionskontrollen jedes Meßkopfes zugeführt werden.

6. Gasdetektionssystem nach einem der Ansprüche 1-5, dadurch gekennzeichnet, daß die vom Sensor gelieferten analogen Meßdaten vom Mikroprozessor im zugeordneten Meßkopf umgesetzt, linearisiert, korrigiert, in der Temperatur, in der jeweiligen Feuchte- und Luftdruckabhängigkeit, soweit erforderlich, kompensiert und in digitaler Form zusammen mit der Sensorkennung und weiteren Statussignalen der zentralen Auswerteelektronik zugeführt sind.

7. Gasdetektionssystem nach einem der Ansprüche 1-6, dadurch gekennzeichnet, daß zusätzlich zu den Meßdaten und der Kennung der jeweils angeschlossenen Gassensoren die aktuellen Temperaturwerte der Umgebungstemperatur am Meßort durch einen zusätzlichen, am Meßkopf angeordneten Sensor mitgemessen und zur zentralen Auswerteelektronik übertragen werden.

8. Gasdetektionssystem nach einem der Ansprüche 1-7, dadurch gekennzeichnet, daß in dem Speicher (EPROM) der zentralen Auswerteelektronik entsprechende Arbeitsroutinen je empfangener Sensorkennung gespeichert sind.

9. Gasdetektionssystem nach einem der Ansprüche 1-8, dadurch gekennzeichnet, daß zur Kalibrierung von Sensoren eine Kalibrieranordnung vorgesehen ist, die mit eigenen Signalen (Kalibriersignal) bei Ansetzen an den Meßkopf (12) den Kalibriervorgang automatisch einleitet und durchführt, wobei das Kalibriersignal vom Meßkopf empfangen und zur zentralen Auswerteelektronik weitergeleitet wird.

10. Gasdetektionssystem nach Anspruch 9, dadurch gekennzeichnet, daß die Kalibrieranordnung (25) zur automatischen Kalibrierung von Sensoren eine Kalibrierkappe (26) aufweist, die einen Eichgas-Aufleitungsbereich zum gassensitiven Element jedes Sensors umfaßt und das Kalibriersignal einer Empfangseinheit am Meßkopf dann zuführt, wenn die Kalibrierkappe sich in Eichposition befindet und das jeweilige Eichgas zugeführt wird.

11. Gasdetektionssystem nach Anspruch 10, dadurch gekennzeichnet, daß die Kalibrierkappe (26) den Meßkopf und die mit diesem verbundene zentrale Auswerteelektronik mit einem spezifischen, den jeweiligen Sensor (S1, S2, T) betreffenden Kalibriersignal beaufschlagt.

12. Gasdetektionssystem nach Anspruch 9, 10 oder 11, dadurch gekennzeichnet, daß das Kalibriersignal ein mit vorgegebener Frequenz gepulstes Infrarotsignal ist, welches von einer Sendediode (36) erzeugt und einer Empfangsdiode (D1, D2) im Meßkopf dann zugeführt wird, wenn ein Strömungssensor in der Kalibrierkappe (26) das Vorhandensein des Prüfgases und ein Kontaktsensor (34) oder ein von der Bedienungsperson betätigter Schalter die Anlage an den jeweiligen, zu kalibrierenden Sensor signalisieren.

13. Gasdetektionssystem nach Anspruch 12, dadurch gekennzeichnet, daß der Strömungssensor ein NTC-Widerstand ist, der in Reihe mit dem die Anlage der Kalibrierkappe signalisierenden Kontaktsensor (Schaltstift 34) liegt und die Sendediode (36) zur Abgabe des Kalibriersignals aktiviert.

14. Gasdetektionssystem nach einem der Ansprüche 9-13, dadurch gekennzeichnet, daß die zentrale Auswerteelektronik nach Empfang des Kalibriersignals und der Kennung des betreffenden Sensors ein Kalibrier-Bestätigungssignal dem jeweiligen Meßkopf zuführt, daß der Meßkopf der Bedienungsperson ein Kalibrierungs-Bestätigungssignal vermittelt und daß die zentrale Auswerteelektronik den Meßwert abspeichert und das Ende des Kalibriervorgangs durch Beeinflussung des Kalibrier-Betätigungssignals anzeigt.

15. Gasdetektionssystem nach Anspruch 14, dadurch gekennzeichnet, daß die zentrale Auswerteelektronik in der Kalibrierphase den vom jeweils mit dem Eichgas beaufschlagten Sensor eingehenden Meßwert auf konstantes Verhalten über der Zeit für einen vorgegebenen Zeitraum überprüft und die neuen Eichwerte bei einer erfaßten Konzentration des Prüfgases (Nullgas) als Nullwert bzw. einer einen vorgegebenen Wert überschreitenden Konzentration des Prüfgases als Steigungswert (Empfindlichkeit) vorgegebener Größe speichert und übernimmt.

16. Gasdetektionssystem nach Anspruch 15, dadurch gekennzeichnet, daß beim Kalibrieren ein Meßsignal unter einer vorgegebenen Schwelle als dem Nullgas zugehörig und ein Meßsignal oberhalb dieser oder einer zweiten Schwelle als Eichgas bekannter Konzentration interpretiert wird.

17. Gasdetektionssystem nach Anspruch 1, dadurch gekennzeichnet, daß bei der Kommunikation die jeweiligen Meßköpfe von der zentralen Auswerteelektronik aufeinanderfolgend in einem bestimmten Ablauf adressiert und zusammen mit der Kennung des jeweiligen Sensors dessen aufbereiteter Meßwert (zusammen mit sonstigen Statussignalen) übernommen wird.

# Fig.1

# Fig.2

Sensorbaugruppe
komplett

Sensorbasisteil

Sensorgehäuse

Sensoreinheit
steckbar

Sensorelektronik

Sensor

O        TOX        UEG        IRA

Fig.3